Europäisches Patentamt

European Patent Office

Office européen des brevets

(19)

(11) EP 0 857 298 B1

(12) EUROPÄISCHE PATENTSCHRIFT

(45) Veröffentlichungstag und Bekanntmachung des Hinweises auf die Patenterteilung:
06.06.2001 Patentblatt 2001/23

(21) Anmeldenummer: 96934629.5

(22) Anmeldetag: 11.10.1996

(51) Int Cl.⁷: $G01N\ 15/02$, G01N 33/48

(86) Internationale Anmeldenummer:
PCT/EP96/04416

(87) Internationale Veröffentlichungsnummer:
WO 97/16718 (09.05.1997 Gazette 1997/20)

(54) **VERFAHREN ZUR DIAGNOSE EINER MALIGNEN ERKRANKUNG ODER DEREN VORLÄUFERSTADIEN**

METHOD OF DIAGNOSING A MALIGNANT DISORDER OF ITS PRECURSOR STAGES

PROCEDE DE DIAGNOSTIC D'UNE PATHOLOGIE MALIGNE OU DE SES STADES PRECURSEURS

(84) Benannte Vertragsstaaten:
**AT BE CH DE FR GB LI NL**

(30) Priorität: **27.10.1995 DE 19540006**

(43) Veröffentlichungstag der Anmeldung:
**12.08.1998 Patentblatt 1998/33**

(73) Patentinhaber: **Forschungszentrum Karlsruhe GmbH**
**76133 Karlsruhe (DE)**

(72) Erfinder:
- **SKOBELTZIN, Evguenia**
  **D-76133 Karlsruhe (DE)**
- **KROUGLIKOV, Ilya**
  **D-76344 Eggenstein-Leopoldshafen (DE)**

- **KNEDLITSCHEK, Gudrun**
  **D-76229 Karlsruhe (DE)**
- **WEIBEZAHN, Karl-Friedrich**
  **D-76297 Stutensee (DE)**
- **DERTINGER, Hermann**
  **D-69126 Heidelberg (DE)**

(56) Entgegenhaltungen:
**US-A- 4 788 155**

- **SURGERY, Bd. 109, Juni 1991, Seiten 747-755, XP000612216 K.M. RIGG ET AL: "Alterations in circulating lymphocyte number and function after circulation through colorectal carcinomas" in der Anmeldung erwähnt**

## Beschreibung

Die Erfindung betrifft ein Verfahren zur Diagnose einer malignen Erkrankung oder deren Vorläuferstadien gemäß dem ersten Patentanspruch.

Zur Diagnose maligner Erkrankungen werden neben bildgebenden Verfahren (Röntgen, Ultraschall, Computertomographie, Kernspintomographie) auch histologische Untersuchungen von Biopsiematerial, Antikörpertests und Tests auf mehr oder weniger spezifische Tumormarker herangezogen. Die zuletzt genannten Tests werden sowohl an histologischen Präparaten als auch mit Blutserum durchgeführt. Die Tumormarker sind größtenteils sehr spezifisch nur für bestimmte Tumorarten indikativ.

Die Hauptprobleme bestehen darin, daß diese Verfahren je nach Stadium der Erkrankung häufig eine unbefriedigende Trefferquote aufweisen und nur ungenügend zwischen malignen und benignen Formen bzw. Vorläuferstadien unterscheiden können. Einige bildgebende Verfahren sind mit einer zum Teil nicht unerheblichen Strahlenbelastung verbunden; die Biopsie stellt einen operativen Eingriff dar, der die Gefahr einer Streuung von Tumorzellen in sich birgt. Darüberhinaus sind die Verfahren meist relativ teuer und in manchen Fällen liegen einigermaßen verläßliche Ergebnisse erst nach einer Tage dauernden Auswertung vor.

Die US-A-4 788 155 offenbart ein Verfahren zum Diagnostizieren der Anwesenheit oder Abwesenheit einer Krankheit, insbesondere eines Tumors, wobei das Verfahren im wesentlichen folgende Schritte umfaßt:

- Zählen der Anzahl gegebener Blutzellen in einer ersten Blutprobe,
- Mischen eines fremden Stoffes, der für die Krankheit spezifisch ist, mit einer zweiten Blutprobe, wodurch die Blutzellen mit dem fremden Stoff reagieren,
- Zählen der Anzahl nicht reagierter und reagierter Blutzellen in der Mischung und
- Vergleichen der Anzahl der in der ersten Blutprobe gezählten Blutzellen mit der Anzahl der in der Mischung gezählten nicht reagierten und reagierten Blutzellen, um dadurch die Diagnose zu stellen.

In *Surgery, Vol 109 (1991) S. 747 - 755* wird über eine Studie berichtet, in der der Einfluß eines Dünndarmtumors auf die durch ihn zirkulierenden Lymphozyten bestimmt werden sollte. An der Studie nahmen Patienten mit einem Dünndarmtumor und als Kontrollgruppe Patienten mit gutartigen Dünndarmerkrankungen und nephrotischen Defekten teil. Alle Patienten wurden operiert. Den tumorkranken Patienten wurde arterielles und venöses Blut aus dem Gefäßsystem des Tumors entnommen. Den Patienten der Kontrollgruppe wurde ebenfalls arterielles und venöses Blut abgenommen, das aus großen Gefäßen des Dünndarms stammte. Die Lymphozyten wurden entweder nach einer Zentrifugierung oder auf der Basis ihres charakteristischen Volumens abgetrennt. Der prozentuale Anteil jeder Lymphozyten-Sub-population wurde ermittelt. Über ein Diagnoseverfahren wird nicht berichtet, da Diagnose bereits vor der Operation bekannt war.

In *Cytometry, Vol. 13 (1992) S. 766 - 774* wird ein Verfahren zur Erkennung und Unterscheidung von Zellen aus Blutausstrichen beschrieben.

Aufgabe der Erfindung ist es, ein weiteres für den Patienten nicht zusätzlich belastendes Diagnoseverfahren für maligne Erkrankungen und deren Vorläuferstadien zu finden, das nicht auf einer Bildgebung beruht und in einem Labor einfach und daher kostengünstig durchführbar ist.

Die Lösung der Aufgabe ist im ersten Patentanspruch beschrieben. Der zweite Patentanspruch beschreibt eine bevorzugte Ausgestaltung des Verfahrens.

Beim erfindungsgemäßen Verfahren werden periphere Blutzellen, vorzugsweise die Lymphozytenpopulation untersucht. Diese Zellen werden dem Organismus zu einen aus arteriellem und/oder kapillärem Blut entnommen. Das arterielle Blut kann einer peripheren Arterie entnommen werden. Anstelle von arteriellem Blut eignet sich in gleicher Weise Kapillarblut, z. B. aus der Fingerbeere oder dem Ohrläppchen des Patienten. Zusätzlich wird dem Patienten venöses Blut, etwa aus einer Armvene, entnommen.

Sowohl das arterielle und/oder das Kapillarblut als auch das venöse Blut werden außerhalb des Körpers untersucht. Zur Untersuchung eignet sich ein Standartblutausstrich. Bestimmt werden die Größenverhältnisse der Zellen, die nachfolgend einer mathematischen Analyse unterzogen werden.

Zur Durchführung des Verfahrens werden nach Blutentnahme einerseits aus einer Arterie und/oder einem kapillaren Blutgefäß und andererseits aus einer Vene vorzugsweise Ausstriche auf Mikroskopobjektträgern angefertigt. Die Blutzellen können nach einem Standardverfahren getrocknet, fixiert und gefärbt werden.

Danach wird ein Größenhistogramm arterieller und/oder kapillärer (Index: A) und venöser (Index: V) Blutzellen, vorzugsweise der Lymphzytenpopulation erstellt. Dies kann durch mikroskopische Ausmessung von Durchmessern an ca. 200 Blutzellen, insbesondere an ca. 200 Lymphozyten erfolgen. Hierzu wird der diskrete prozentuale Anteil $(N_i)^A$ bzw. $(N_i)^V$ in einem Größenbereich von 6 Um und 16 μm mit einer Auflösung von 1 μm erfaßt, so daß zu jeder Zellgröße im Bereich von 6 μm und 16 μm sowohl im arteriellen oder kapillärem $(^A)$ als auch im venösen Blut $(^V)$ der prozentuale Anteil $(N_i)^A$ bzw. $(N_i)^V$ für i = 6 μm bis i = 16 μm an der Gesamtzahl der jeweiligen Blutzellart ermittelt ist.

Lymphozyten < 6 µm existieren im allgemeinen nicht.

Die Auswertung der Messungen wird durch zwei Figuren veranschaulicht.

Die Fig. 1 zeigt das Diagramm $(N_i)^A$ bzw. $(N_i)^V$ gegen i.

Fig. 2 stellt ein Koordinatensystem dar, das die Stellung einer Diagnose zuläßt.

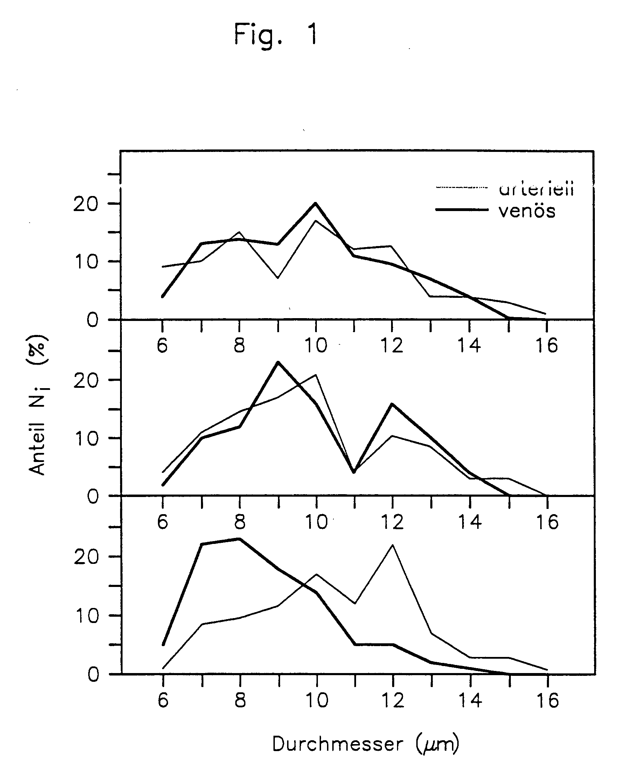

Wie in Fig. 1 gezeigt können die Werte für $(N_i)^A$ bzw. $(N_i)^V$ zur Veranschaulichung graphisch gegen die Zellgröße i (6 µm $\leq$ i $\leq$ 16 µm) aufgetragen werden. In der Figur sind konkrete, an verschiedenen Patientinnen ermittelte Werte für $N_i$ in einem Diagramm dargestellt. Der obere Teil der Figur bezieht sich auf die Ergebnisse an einer gesunden Probandin; der mittere Abschnitt zeigt die Ergebnisse für eine Patientin mit Fibromyom und der untere Abschnitt die Ergebnisse für eine Patientin mit Ca. corporis uteri Stadium II. Ein Arzt mit umfassender Erfahrung könnte mit solchen Diagrammen das Bestehen einer malignen Erkrankung oder deren Vorläuferstadien diagnostizieren bzw. eine solche Erkrankung ausschließen. Eindeutigere Ergebnisse werden jedoch erhalten, wenn die Werte für $(N_i)^A$ und $(N_i)^V$ gewichtet und gemittelt werden.

Hierzu werden sowohl die Werte $(N_i)^A$ als auch die Werte $(N_i)^V$ mit einem Wichtungsfaktor $\alpha$ multipliziert. Der Wichtungsfaktor beträgt vorzugsweise $2^{10}$ für i = 6 µm; für i = 6 + n µm wird der Exponent des Wichtungsfaktors vorzugsweise um n vermindert. In jedem Fall nimmt der Wert des Wichtungsfaktors mit steigender Zellgröße ab. Geeignete Wichtungsfaktoren $\alpha$ sind in der folgenden Tabelle zusammengestellt.

Tabelle:

| | |
|---|---|
| für i = 6 µm | $\alpha = 2^{10} = 1024$ |
| für i = 7 µm | $\alpha = 2^9 = 512$ |
| für i = 8 µm | $\alpha = 2^8 = 256$ |
| für i = 9 µm | $\alpha = 2^7 = 128$ |
| für i = 10 µm | $\alpha = 2^6 = 64$ |
| für i = 11 µm | $\alpha = 2^5 = 32$ |
| für i = 12 µm | $\alpha = 2^4 = 16$ |
| für i = 13 µm | $\alpha = 2^3 = 8$ |
| für i = 14 µm | $\alpha = 2^2 = 4$ |
| für i = 15 µm | $\alpha = 2^1 = 2$ |
| für i = 16 µm | $\alpha = 2^0 = 1$ |

Anschließend werden die Summen

$$A = \Sigma \; \alpha_i \cdot (N_i)^A$$

$$V = \Sigma \; \alpha_i \cdot (N_i)^V$$

gebildet. Aus den Summen A und V werden die Linearkombinationen

$$X = A + V$$

und

$$Y = A - V$$

berechnet.

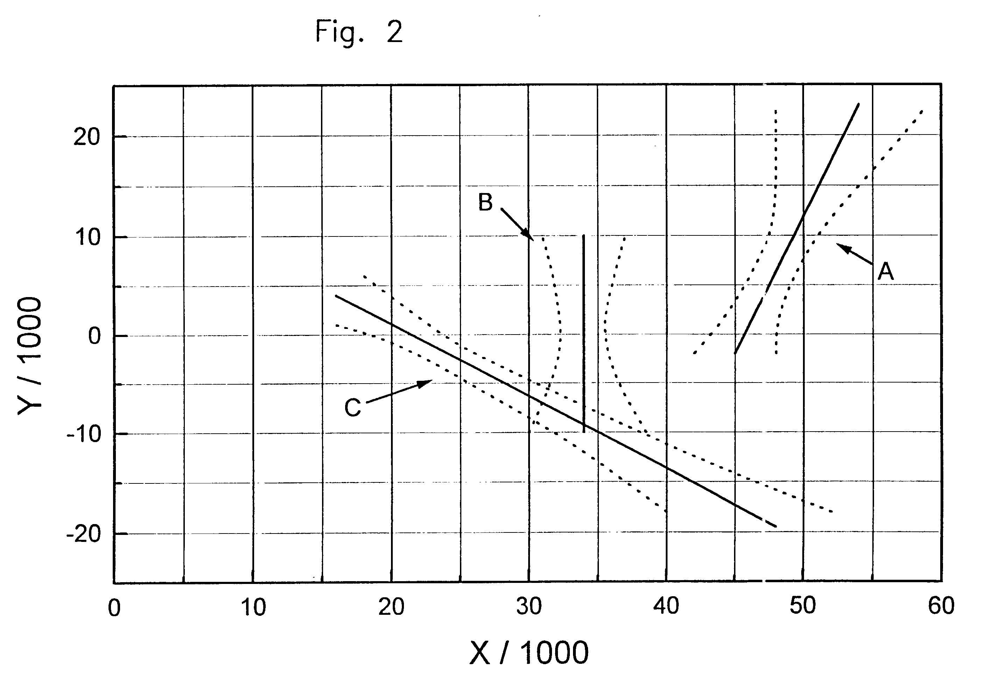

Wie Fig. 2 zeigt, lassen sich die Werte X und Y als Punktkoordinaten in einer Ebene darstellen. Die Darstellung in einem Koordinatensystem erlaubt sofort die diagnostische Zuordnung, da sich bei der Untersuchung einer größeren Anzahl von Patientinnen eine Ausbildung von Koordinatenbereichen für die unterschiedlichen Krankheitsbilder ergeben hat. Die Bereiche "gesund" (a) über die benigne Form oder Vorstufe (Fibromyom) (b) bis hin zur malignen Form (Carcinoma corporis uteri unterschiedlichen Schweregrads) (c) sind im Koordinatensystem deutlich abgrenzbar. Die Daten für Fig. 2 wurden anhand von Untersuchungen an einer ausländischen Population gewonnen. Die durchgezogenen Linien stellen die Mittelwerte, die gestrichelten Linien die 95%-Mutungsbereiche dar.

Die Erfindung weist den Vorteil auf, daß die Diagnose anhand einer Blutentnahme, die in der Regel ohnehin erfolgt, vorgenommen werden kann. Das erfindungsgemäße Verfahren stellt daher für den Patienten keine Belastung dar. Die Trefferquote des Verfahrens liegt bei über 95 %, wie Vorversuche belegt haben. Teure Apparaturen sind zur Durchführung nicht erforderlich, so daß das Verfahren auch in Regionen ohne hochtechnisierte Versorgung durchführbar ist. Die Ermittlung kann manuell an einem Mikroskop vorgenommen werden; eine Automatisierung mit einer preiswerten Vorrichtung erscheint möglich. Schließlich eignet sich das erfindungsgemäße Verfahren wegen seiner geringen Kosten auch zur Vorsorgeuntersuchung in größerem Umfang.

Die Erfindung wird im folgenden anhand eines Durchführungsbeispiels näher erläutert.

Aus Standardblutausstrichen arteriellen und venösen Blutes dreier Patientinnen werden die prozentualen Anteile ($N_i$) für jede Lymphozytengröße im Bereich von 6 µm bis 16 µm unter dem Mikroskop bestimmt. Das Größendiagramm ist in der Figur dargestellt.

Die Werte für $(N_i)^A$ und $(N_i)^V$ werden mit dem jeweiligen Wichtungsfaktor multipliziert und aufsummiert. Für die Linearkombinationen X = A + V und Y = A - V erhält man folgende Werte:

| Kontrollwert (gesunde Probandin) | x = 40762, | y = +3200 |
| Patientin mit Fibromyom | x = 32810, | y = +2538 |
| Patientin mit Ca. corp. ut. II | x = 39361, | y = -15927. |

Auffallend ist insbesondere der hohe negative Wert für y bei der Patientin mit Ca. corporis uteri Stadium II.

## Patentansprüche

1.   Verfahren zur Diagnose einer malignen Erkrankung oder deren Vorläuferstadien, bei dem

a) zwei Blutproben eines Patienten bereitgestellt werden, von denen eine arterielles und/oder kapilläres (Index: A) und die andere venöses (Index: V) Blut enthält,

b) jeweils die prozentualen Anteile $N_i$ an Blutzellen mit einer Auflösung von 1 µm in einem Größenbereich i = 6 µm bis i = 16 µm bezogen auf die Gesamtblutzellenanzahl sowohl im arteriellen und/oder kapillären [$(N_i)^A$] als auch im venösen Blut [$(N_i)^V$] bestimmt werden,

c) die für $N_i$ erhaltenen Werte jeweils mit einem Wichtungsfaktor $\alpha_i$, dessen Wert bei einer steigenden Größe der Blutzellen abfällt, multipliziert werden,

d) die Summen

$$A = \Sigma\ \alpha_i \cdot (N_i)^A$$

$$V = \Sigma\ \alpha_i \cdot (N_i)^V$$

und hieraus die Werte X = A + V und Y = A - V berechnet werden und

d) die Abweichung der aus den Blutproben des Patienten ermittelten Werte X und Y von aus Blutproben eines gesunden Probanden erhaltenen Vergleichswerten für X und Y bestimmt wird.

2.   Verfahren nach Anspruch 1, bei dem als Blutzellen Lymphozyten verwendet werden und als Gesamtblutzellenanzahl die Lymphozytenanzahi eingesetzt wird.

3.   Verfahren nach Anspruch 3, bei dem die Wichtungsfaktoren $2^{10}$ für i = 6 µm, $2^9$ für i = 7 µm, $2^8$ für i = 8 µm usw. bis $2^0$ für i = 16 µm betragen.

## Claims

1.   Method of diagnosing a malignant illness or the early stages thereof, wherein:

a) two blood samples of a patient are prepared, one of which contains an arterial and/or capillary (Index: A) blood, and the other contains venous (Index: V) blood,

b) the $N_i$ percentage proportions of blood cells, having a dissolution of 1 μm in a range of magnitude of between i = 6 μm and i = 16 μm, relative to the total number of blood cells, are determined both in the arterial and/or capillary blood $[(N_i)^A]$ and in the venous blood $[(N_i)^V]$,

c) the values obtained for $N_i$ are each multiplied with a weighting factor $\alpha_i$, the value of which decreases with a rising magnitude of the blood cells,

d) the sums

$$A = \Sigma \; \alpha_i \cdot (N_i)^A$$

$$V = \Sigma \; \alpha_i \cdot (N_i)^V$$

and the values X = A + V and Y = A - V are calculated therefrom, and

e) the deviation of the values X and Y, ascertained from the blood samples of the patient, are determined from comparative values for X and Y, which are obtained from blood samples of a healthy test person.

2. Method according to claim 1, wherein lymphocytes are used as the blood cells, and the number of lymphocytes is used as the total number of blood cells.

3. Method according to claim 3, wherein the weighting factors are $2^{10}$ for i = 6 μm, $2^9$ for i = 7 μm, $2^8$ for i = 8 μm, etc. down to $2^0$ for i = 16 μm.

**Revendications**

1. Procédé de diagnostic d'une pathologie maligne ou de ses stades précurseurs,
caractérisé en ce qu'

a) on met à disposition deux échantillons de sang d'un patient, dont l'un contient un sang artériel et/ou capillaire (index A) et l'autre un sang veineux (index V),

b) on détermine à chaque fois la proportion en pourcentage $N_i$ de cellules sanguines ayant une résolution de 1 μm dans un domaine de tailles i = 6 μm à i = 16 μm rapporté au nombre de cellules sanguines total, aussi bien dans le sang artériel et/ou capillaire $[(N_1)^A]$ que dans le sang veineux $[(N_i)^V]$,

c) les valeurs obtenues pour $N_i$ sont multipliées respectivement par un facteur de pondération $\alpha_i$, dont la valeur décroît pour une taille croissante des cellules du sang,

d) on calcule les sommes :

$$A = \Sigma \; \alpha_i \cdot (N_i)^A$$

$$V = \Sigma \; \alpha_i \cdot (N_i)^V$$

et à partir de cela les valeurs X = A + V et Y = A - V, et

e) on détermine la différence des valeurs X et Y déterminées à partir des échantillons de sang du patient, avec des valeurs de comparaison obtenues à partir d'échantillons de sang d'un volontaire sain.

2. Procédé selon la revendication 1,
caractérisé en ce qu'
on utilise comme cellules sanguines des lymphocytes, et comme nombre total de cellules on utilise le nombre de lymphocytes.

3. Procédé selon la revendication 1,
caractérisé en ce que
les facteurs de pondération s'élèvent à $2^{10}$ pour i = 6 μm, à $2^9$ pour i = 7 μm, $2^8$ pour i = 8 μm etc... jusqu'à $2^0$ pour i = 16 μm.

# Fig. 1

Fig. 2